# EUROPEAN PATENT SPECIFICATION

(11) **EP 3 587 561 B1**
(45) Date of publication and mention of the grant of the patent: **09.04.2025**
(21) Application number: 18757464.5
(22) Date of filing: 22.02.2018
(51) Int. Cl.: C12N 5/076, A61B 17/425, A61D 19/00, C12N 5/071, A61B 17/43, B01D 29/085, C12M 1/12, G01N 1/34

(54) **DEVICE AND METHOD FOR SEPARATING MOBILE CELLS**
VORRICHTUNG UND VERFAHREN ZUR TRENNUNG MOBILER ZELLEN
DISPOSITIF ET PROCÉDÉ DE SÉPARATION DE CELLULES MOBILES

(30) Priority: 24.02.2017 AR P170100483
(43) Date of publication of application: 01.01.2020
(73) Proprietor: Selectivity SAS, Córdoba (CP: 5000) (AR)
(72) Inventor: REPETTO, Herberto Ernesto Héctor, Caba, 1431 (AR)
(74) Representative: Trupiano, Federica
(86) International application number: PCT/ES2018/070135
(87) International publication number: WO 2018/154169

(56) References cited:
- WO-A1-02/102257
- WO-A1-2013/040428
- US-A1- 2014 051 112
- US-A1- 2016 017 273
- REZA NOSRATI ET AL: "Rapid selection of sperm with high DNA integrity", LAB ON A CHIP, vol. 14, no. 6, 1 January 2014 (2014-01-01), pages 1142, XP055635383, ISSN: 1473-0197, DOI: 10.1039/c3lc51254a
- DONG-HOON CHOI ET AL: "Fabrication of a membrane filter with controlled pore shape and its application to cell separation and strong single cell trapping", JOURNAL OF MICROMECHANICS AND MICROENGINEERING, vol. 25, no. 10, 9 September 2015 (2015-09-09), GB, pages 1 - 11, XP055367342, ISSN: 0960-1317, DOI: 10.1088/0960-1317/25/10/105007
- YADONG TANG ET AL: "Microfluidic device with integrated microfilter of conical-shaped holes for high efficiency and high purity capture of circulating tumor cells", SCIENTIFIC REPORTS, vol. 4, no. 1, 13 August 2014 (2014-08-13), XP055660349, DOI: 10.1038/srep06052
- SEO, DB ET AL.: "Development of sorting, aligning, and orienting motile sperm using microfluidic device operated by hydrostatic pressure", MICROFLUIDICS AND NANOFLUIDICS, vol. 3, no. 5, October 2007 (2007-10-01), pages 561 - 570, XP019522724

## Description

### TECHNICAL FIELD OF INVENTION

The present invention describes a method to separate motile cells from a cell population. It is specially set within spermatozoa retrieval techniques for Assisted Reproductive Technology (ART) in which quality and a quantity of spermatozoa are needed to be retrieved, in relation to their motility, morphology and DNA integrity (1) of without the need of other devices.

### STATE OF THE ART

There are plenty of methods and devices for cell separation in the state of the art. Such methods and devices are based on the characteristic properties of motile cells as spermatozoa, such as rheotaxis (4) among others. Devices and methods for separation in the state of the art use filtering membranes, density gradients, perforated membranes which block the passage of cells, among others.

Patents US6129214 (A) and US6357596B1 describe a method and a device to separate the best spermatozoa using a membrane, where the spermatozoa which penetrate the membrane may be kept in a suitable medium. According to what is mentioned in the description, this device separates motile spermatozoa when they pass through a porous membrane of 5- 8 µm-pore. This device does not separate spermatozoa by rheotaxis but filters non-motile spermatozoa.

Patent application US2008311653 (A1) describes a method and apparatus for regulating optimum flow of semen and separating motile sperms. The device consists of a cylindrical shape container, a membrane comprised of pores (holes of between 10 and 150 µm diameter), and a second cylindrical container which is assembled together with the first The separation method is based on the capacity of spermatozoa to swim upstream (swim-up). The method uses a system of currents which induces rheotaxis, but its realization becomes complex since it requires a degree of specialization and devices that the device of the present invention does not need. The quantity of medium required by this method is higher and it cannot proceed without using an oven. The application WO2008104042A1 describes a spermatic-cell separation process by a 2-3 micron pore filtering membrane. Said membrane may be of various materials such as PES, PVDF, MCE, PTFE, Nylon, polycarbonate, etc. This type of device does not allow the passage of motile cells but withholds them in the membrane pores.

Patent US5575914A describes a sperm filter trap having compressed glass wool filter material to separate low-motility spermatozoa from those of a high motility. It is neither based on rheotaxis; it filters non-motile spermatozoa.

Patent Application CA2834007A1 describes a device for separating sperm which uses a radial array of microchannels disposed so as to direct sperm from a reservoir to another. Said microchannels are between 50 and 300 microns in width, from 100 to 200 microns in height, and between 6 and 9 mm in length. The microchannels are cylindrical in shape. This device is complex in design and manufacture. Spermatozoa do not swim upstream and therefore, the device directs them under the definition of "wall swimming", i.e., the shape of the channels redirects spermatozoa. In addition, this device needs a stove. On the contrary, the present invention achieves self-selection taking advantage of the spermatozoa's ability to penetrate and swim upstream, since an initial stream is generated within the device towards the site where the sperm is to be placed. Also, the channel shape of the present invention achieves extraordinary yields without requiring additional devices.

Application US2016017273 discloses a method and a device to separate spermatozoa applying the swim-up technique.

WO 02/102257 A1 discloses a sperm separation device comprising a tubular conduit defining an upstream zone for receiving semen and a downstream zone from which sperm are arranged to be harvested. The upstream and downstream zones are separated by constriction means within the conduit, the constriction means being arranged to establish capillary flow of a fluid medium from the upstream zone to the downstream zone, whereby motile sperm in semen inserted into the conduit upstream from the constriction means and subjected to the flow of the fluid medium are enabled, having passed through the constriction means, to maintain a position in the downstream zone for harvesting.

WO 2013/040428 A1 discloses a microfluidic chip for acquiring sperms with high motility in a sperm sample, which comprises: (a) an inlet region at one end of the microfluidic chip; (b) a first flow channel that is in fluidic communication with the inlet region; (c) a divergent channel, which is arranged at the downstream of the flow channel and in fluidic communication with the flow channel; and (d) one or more outlet region(s) located at one or both sides of the divergent channel. According to WO 2013/040428 A1, the sperm sample flows from the flow channel to the divergent channel, and that the divergent channel comprises one or more outlet region(s) located at one or both sides of the divergent channel to grade sperms with various motility scales.

Reza Nosrati et al. "Rapid selection of sperm with high DNA integrity", LAB ON A CHIP, vol. 14, no. 6, 1 January 2014, page 1142, XP055635383, describes a device for sorting sperm cells based on the progressive motility in 500 parallel microchannels. During separation no flow is present.

Dong-Hoon Choi et al. "Fabrication of a membrane filter with controlled pore shape and its application to cell separation and strong single cell trapping", JOURNAL OF MICROMECHANICS AND MICROENGINEERING, vol 25, no. 10, 9 September 2015, pages 1-11, XP055367342, discloses a membrane filter with controlled (conical) pore shape and its application to cell separation and single cell trapping. Dong-Hoon Choi et al. describes that the membrane with conical shape pores shows strong potential as an analytical tool for a single cell analysis. The fabricated conical pores remarkably enhance the trapping ability, and thus the whole process, including filtration, single cell isolation, antibody staining, immunofluorescence imaging, and *in situ* hybridization, can be easily performed on the membrane after the filtration process thanks to the strong trapping ability.

Yadong Tang et al. "Microfluidic device with integrated microfilter of conical-shaped holes for high efficiency and high purity capture of circulating tumor cells", SCIENTIFIC REPORTS, vol. 4, no. 1, 13 August 2014, XP055660349, discloses a microfluidic device for high efficiency and high purity capture of CTCs (circulating tumor cells) and the integration of a microfilter with conical-shaped holes and a micro-injector with crossflow components for size dependent capture of tumor cells without significant retention of non-tumor cells.

The devices and methods present in the state of the art have technical disadvantages compared with the present invention since the medium used for the separation can become clogged, they require manipulation by someone relatively skilled in laboratory instrument handling and therefore it is also necessary handling in controlled environments, making it impossible their use on the field or in consulting rooms. On the other hand, most of them require extra filtering and/or centrifugation operations, favouring the likelihood of sample contamination, material loss, DNA alteration, etc., whether due to the increased sample manipulation time or pressure caused by revolutions during centrifugation.

The present invention provides a method for the separation of motile cells which does not require the operations of filtration and/or centrifugation; its manipulation is simple and achieves excellent results of separation of those motile cells of a cell population, including the separation of motile cells with damaged DNA. A remarkable feature of the present invention is that it does not require an oven to perform motile cell separation. This allows taking the necessary actions for artificial insemination in humans as well as in animals under basic conditions, without the need of the usual equipment regarding this type of techniques. The method of the present invention even allows on-field artificial insemination.

### BRIEF DESCRIPTION OF THE INVENTION

The method of the present invention for the separation of motile cells from a cell population, preferably spermatozoa, uses a device which comprises a first reservoir and a second reservoir linked by at least a membrane, characterized in that said membrane comprises a multiplicity of channels with at least one end showing a reduction in its diameter towards the interior of said channels. And where said membrane is made preferably of a material selected from the group comprising of glass, PET, metal, polymer, carbon fiber and the mixtures or combinations thereof. Where said channels comprise preferably in at least one of their ends, a shape selected from the group comprised of conical and hyperboloid shapes, and said membrane comprises a multiplicity of channels. In a preferred embodiment, said membrane is a perforated plate.

In addition, said channel comprises, in another preferred embodiment, both ends of shapes selected from the group comprised of conical, paraboloid and hyperboloid shapes.

Said membrane comprises a multiplicity of channels which allow the separation of motile spermatozoa on one side of said membrane and of the elements or non-motile cells which remain on the other side of the membrane.

Preferably, said motile cells separation device used in the method of the invention has channels comprising a first outer diameter of between 50 and 200 µm; an inner diameter of between 8 and 15 µm, and length of between 50 and 600 µm; more preferably they comprise a first outer diameter of between 50 and 200 µm and a second outer diameter of between 10 and 50 µm; an inner diameter of between 8 and 15 µm and a length of between 50 and 350 µm.

Also, the motile cells separation device used in the method of the present invention presents preferably said membrane with between 1,000 and 20,000 channels /cm²; and preferably the volume of said first reservoir is between 1 and 5 ml, and the volume of said second reservoir is between 0.5 and 1.5 ml; and where said second reservoir comprises a higher level than said first reservoir. Also, said second reservoir is filled with a culture medium generating a current within said culture medium towards said first reservoir.

Where said membrane is made of a material selected from the group comprising of glass, PET, metal, ceramics, polymer, carbon fiber and the mixtures or combinations thereof.

In some embodiments, the motile cells separation method of the present invention allows achieving a decrease of sperm with damaged DNA in said second reservoir regarding a sample of fresh semen. The present invention succeeds in reducing up to nine times the value of the TUNEL Test (DNA damage) compared with the direct method. In particular, a decrease of at least 3 times is observed for the invention realized in PET (from 35% to 10%, for sample D, table 1); and up to 9 times for the invention in glass (from 35% to 4% for the same sample D). In addition, the methodof the present invention increases the quantity of spermatozoa with normal morphology in at least 80% (morphology values for Sample D).

Object of the present invention is a method to separate motile cells from a cell population according to claim 1.

Where said separation method for motile cells from a cell population in step "b" comprises a counter-current in a culture medium flowing in the opposite sense of the passage of said motile cells, where said current is generated by pressure difference, or capillarity, and said pressure difference is caused by the difference in level between the reservoirs located at both sides of said membrane. In addition, said step "c" comprises incubating for a period of between 1 and 90 minutes at a temperature ranging from 35 to 37ºC. Preferably between 10 and 90 minutes. And, the method of the present invention uses the device of the present description.

### BRIEF DESCRIPTION OF THE FIGURES

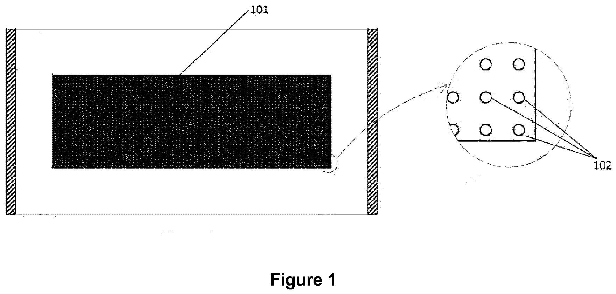

FIGURE 1: Cross section, Front view showing a membrane (101), where the enlarged image exhibits the membrane channels (102).

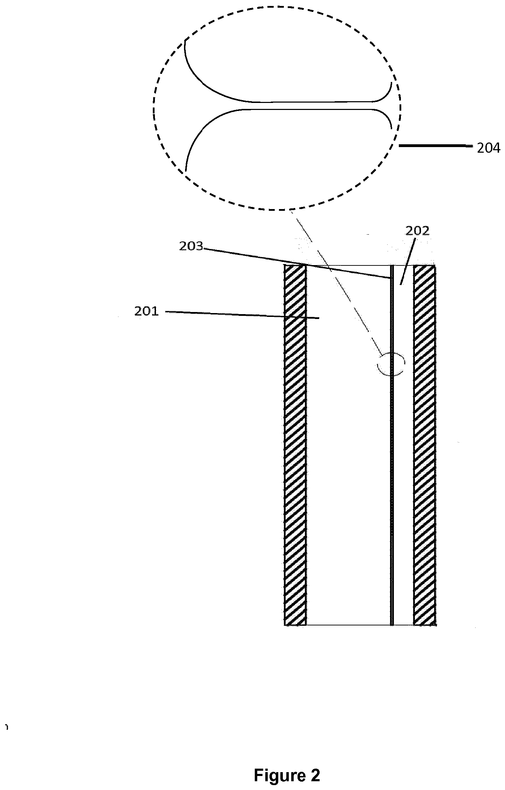

FIGURE 2: Side cross section of the device showing the first reservoir (201), the membrane (203) and the second reservoir (202). It also shows the enlarged view of the preferred shape of the type of membrane channel is also shown.

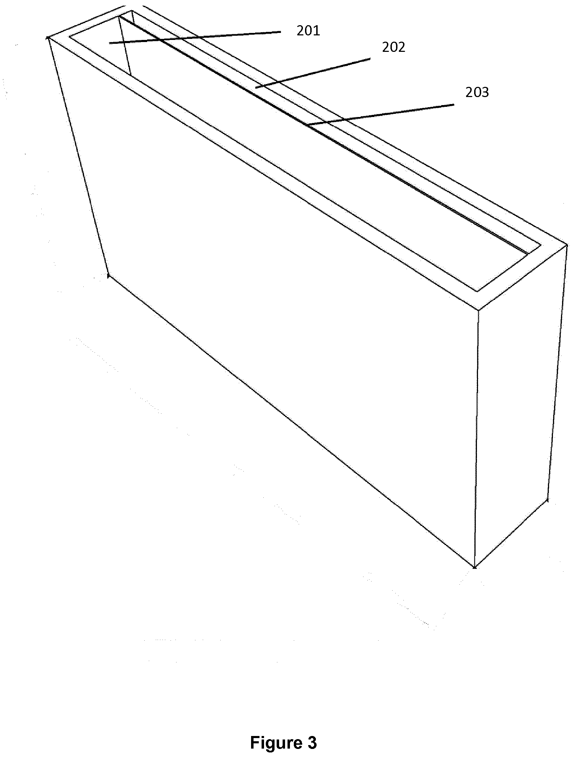

FIGURE 3: Complete possible scheme of the device used in the method of the present invention showing the first reservoir (201), the membrane (203) and the second reservoir (202).
FIGURE 4 represents the device adapted to the intracytoplasmic sperm injection (ICSI) technique.
FIGURE 5 shows schematic views of some of the shapes that the membrane channels of the present invention can adopt.
FIGURE 6 shows an alternative embodiment of the device used in the method of the present invention.

### DETAILED DESCRIPTION OF THE INVENTION

According to the present invention, a cell population comprises a fluid which may be a culture medium, a biological fluid with a great variety of cells among which there are cells with locomotory capacity, hereinafter called motile cells. The example of the present invention comprises the separation of motile cells of the spermatic type; however, the device and method also apply to other types of motile cells such as microorganisms or parasites.

According to the present invention, membrane means any material characterized by containing at least one channel that passes from one face to the other of the membrane and connects both reservoirs of the device used in the method of the present invention.

The present invention describes a device and a method for the separation of motile cells, preferably spermatic cells, which is not based on the premises of swim-up or what is used by most other systems, but it is based on two aspects 1) the search by a gamete or cell of a place to enter, at random, facilitated by the channel shape and 2) the microcurrents generated by the spermatozoa themselves and that end up directing the following ones by rheotaxis (firstly induced by pressure difference and later self-induced by the passage through the narrow channel).

The device used in the method of the present invention does not require any other laboratory element to perform according to its intended use; i.e. no additional tubes, nor centrifuge, no stove required. It does not require electricity, either, which makes it a basic and innovative tool when it comes to extending assisted fertilization world widely, enabling health care professionals and veterinarians to access by themselves to a tool to be used in for example in rural areas far from laboratories. The incubation required by the method of the present invention may be carried out keeping the device within the closed fist of an adult or in contact with the human body.

The device used in the method of the present invention comprises a first reservoir (201), where the semen sample from which spermatozoa are to be separated are placed, a second reservoir (202) to where spermatozoa or motile cells migrate and are collected, being both reservoirs linked by a membrane (203). Said membrane comprises a multiplicity of perforations which constitute channels through which motile cells migrate. Said perforations, called channels, can be of the same or different diameter according to the section measured, i.e., taking into account the section that is linked to the first reservoir, the section that is linked to the second reservoir and the section located between the two sections already mentioned (both called outer sections), called hereinafter middle section.

In a preferred aspect of the present invention, the channels of the membrane are of the same diameter in all the sections of said channels.

In another aspect of the present invention, the diameter of the channels is different in the sections linked to the reservoirs regarding the middle section. In a preferred embodiment of the present invention, said outer sections of said channels are of a greater diameter than the diameter of the middle section of said channel.

In another aspect of the present invention, said channel outer sections are geometric in shape, such as conical, hyperbolic or parabolic.

The device used in the method of the present invention operates in the absence of filtering or the application of electromagnetism, or static currents or immunological labelling methods. The device used in the method of the present invention makes the spermatozoa swim in the same way they would do *in vivo,* migrating in groups from a reservoir passing through a membrane towards the other reservoir, leaving behind detritus, mostly spermatozoa with damaged DNA and non-motile cells. Rheotaxis -the property of having the design and natural behaviour of gametes to penetrate interfaces-is taken advantage of. Said behaviour is observed to a greater extent in spermatozoa with DNA that is not damaged. The device uses the property of rheotaxis of spermatozoa generated by a difference in pressure between the two reservoirs, achieving so due to the higher initial level of the second reservoir (where motile spermatozoa are retrieved), which persists until both pressures are balanced, though they are also involved as the driving forces of said capillary current and possibly difference in concentration in both reservoirs. Beyond the phenomenon caused by the current, it is a fact that there is a fluid current from the second reservoir towards the first one, which generates an impulse for the healthy spermatozoa to swim upstream. Up to that instant, the currents induced through each channel through the culture medium towards said first reservoir for whole semen collection, favours rheotaxis of the spermatozoa which retain such property intact, being the system from that moment (balanced pressure or zero rate flow) self-sustained by the microcurrents induced now by the group of spermatozoa which continues passing through the membrane.

This condition of the most apt spermatozoa to pass through the membrane of the present invention agrees with the results in the significant increase in the percentage of normal morphology and the highly significant decrease in the percentage of damaged DNA. The characteristics of the present invention allow the self-selection among gametes of rapid linear motility, which agree, as it is expected, with a percentage in an increased normal morphology and a higher percentage of DNA without alterations.

In the device used in the method of the present invention, the spermatozoa are following a microcurrent caused by the flow between the compartment of the retrieval medium and the semen compartment, and later self-sustained by those which follow. In other words, after one finds the entrance, the others see their way favoured by the microcurrents caused by the first flagellum, to which the microcurrent of the successive flagella add to, thus enhancing the current and therefore the rheotaxis effect. This way spermatozoa leave the seminal plasma by themselves and access the insemination culture medium, leaving behind in the seminal plasma all types of elements which are not spermatozoa or else non-motile spermatozoa (cells, detritus, immobile spermatozoa and also spermatozoa without rheotaxis capacity), since they do not show the capacity of spermatozoa of penetrating this type of orifice.

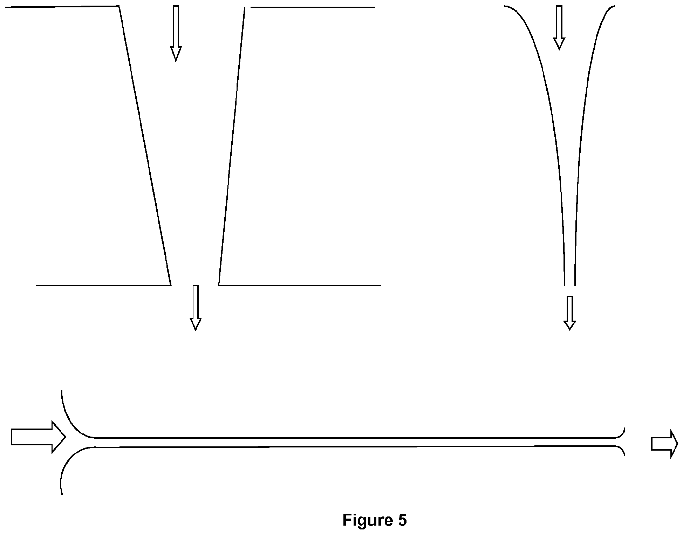

The device used in the method of the present invention comprises at least one membrane with a multiplicity of channels of shapes selected from the following ones: conical, hyperbolic, paraboloid of revolution, among many others with allow widening in at least one of the ends of the channels as it can be seen in Figure 5. The quantity, quality and type of channels vary according to the material used to manufacture the plate. The size of the reservoirs for the semen sample may also vary according to the volume to be processed, among other factors. The manufacturing methods are known in the state of the art and may be performed by laser, among other perforation mechanisms.

In a preferred embodiment of the present invention, the membrane comprises a channel density of between 1,000 and 20,000 per cm² of membrane.

In another aspect of the present invention, the perforations or channels of the membrane of the present invention comprise a length of between 100 and 600 µm.

As it was previously mentioned, the channels of the present invention comprise three sections of different diameters; a first outer diameter linked to said first reservoir (where semen is placed), an inner diameter and a second outer diameter which links the membrane with said second reservoir (where the motile cells are collected in a culture medium supplied to that aim). Thus, said first outer diameter comprises between 50 and 200 µm, said inner diameter comprises between 8 and 15 µm, and said second outer diameter comprises between 10 and 50 µm.

In another aspect of the present invention, the perforations of the membrane comprise different geometric shapes such as cylindrical, conical, parabolic, hyperbolic, hyperboloid of revolution. Preferably, the geometric inlet and outlet perforation shapes is a curve of revolution which may be straight (which generates a cone) or else a curve the type of a parabolic or hyperbolic shape, resulting in a figure of revolution with an outer diameter greater than the inner diameter (204).

Surprisingly, it has been found that the shapes described of said perforations which originate the channels of said membrane, when widened at the inlet favour a configuration which increases the efficiency of the device used in the method of the invention significantly to obtain a higher concentration of viable motile cells.

The reservoirs of the device used in the method of the present invention comprise a volumetric capacity which depends on various factors: the volume of the sample to be processed, the dimensions of the membrane, among others. In a preferred embodiment, the reservoirs used for a membrane of 1x3 cm vary between 0.5 and 4.0 ml. It is vital to highlight that the device used in the method of the present invention generates a current in the fluid or culture medium in such a way that the motile cells, preferably spermatozoa, use their ability to swim upstream for their separation. Said current is caused by a pressure difference in the reservoirs. In order to generate pressure difference, the second reservoir contains a higher liquid level, thus generating a current towards the first reservoir through the membrane. In a preferred embodiment, in the device used in the method of the present invention, the sample to be separated is placed in said first reservoir and fluid or culture medium is loaded into said second reservoir to retrieve motile cells separated from the rest of the population on their passage through said membrane. To generate difference in pressure and therefore cause a current flowing to the first reservoir, first the second reservoir has to be filled to same level or a higher level than said first reservoir; subsequently, the first reservoir is filled with the sample of the population of cells to be separated.

The device used in the method of the present invention separates motile spermatozoa within a period of between 1 and 90 minutes. Preferably between 10 and 90 minutes.

In another aspect of the present invention, the same can be performed in the absence of an stove, since it also works even when it is heated by the warmth released by operators themselves.

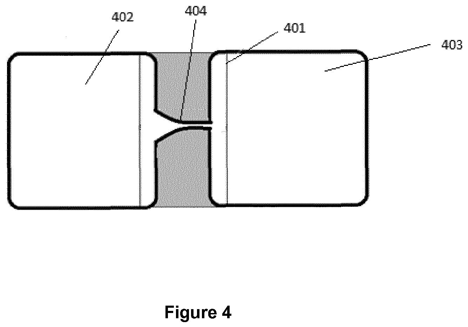

In another aspect of the embodiment of the present invention, the device is adapted to the intracytoplasmic sperm injection (ICSI) technique. For inseminating with semen microinjection into an ovocyte, separating high performing spermatozoa on the same injection membrane may be of use. It is based on an alternative embodiment of the present invention, but with dimensions suitable for the ICSI process. A preferred alternative embodiment not according to the present invention with only one channel that is shown in Figure 4 comprises said two reservoirs, also called micro cuvettes (402 and 403) linked by a channel with a funnel-shaped inlet connecting both reservoirs (404). Both reservoirs are covered by a coverglass (401) thus enabling a 10 µ channel. This system allows retrieval of high quality spermatozoa *in situ.* In this alternative embodiment, the membrane has only one channel.

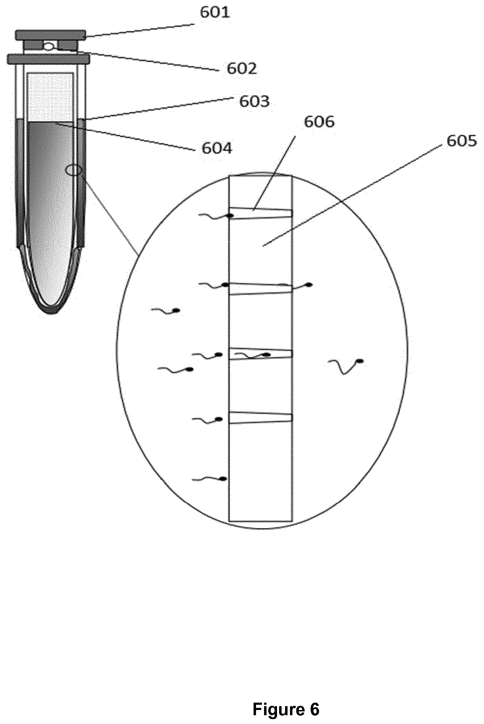

In another alternative embodiment of the present invention, the invention comprises a cylindrical shape as shown in Figure 6, comprising a cover (601), a hole close to the opening of said tube (602), a first reservoir (604) and a second reservoir (603) where it can be seen that the level of the second reservoir, outlet and collection reservoir for the selected spermatozoa is higher than the level of the first reservoir where the semen sample with the spermatozoa to be separated is loaded. Figure 6 shows an enlargement of the section exhibiting the membrane (605) with channels (606) along which spermatozoa flow.

### Examples of Application

### Example 1

### Device with glass membrane Tests D, E and F

**On a glass membrane** The motile cells separation device of the present invention (MXM) comprises two reservoirs plate separated by a 130 ±40 µm thick glass membrane plate with conical channels having the length of the glass width, of about 1mm, a 15 µm-first diameter (inlet) and a 8 µm-second diameter (outlet). In the first place retrieval medium Ham F10 1X with HEPES was placed, supplemented with SSS and Penicillin / streptomycin was placed in the retrieval reservoir (second reservoir); then semen samples D, E y F were placed after having been processed according to WHO2010 Protocol, in the remaining reservoir (first reservoir).

In this example the membrane is 1cm x 2cm, with a density of 6600 perforations/cm2. The first reservoir, where the sample to be processed is placed, has a maximum volumetric capacity of 3mL, and the second reservoir comprises a maximum capacity of 1mL.

### Example 2

### Device with PET membrane Tests A, B, C and D

The membrane made of PET was made with 2500 channels, each 200µm in length. Conical channels comprise a first (inlet) diameter of 15 µm and a second (outlet) diameter of 10 µm. The PET membrane is 1cm x 2cm. The reservoirs are the same ones used for the glass membrane in example 1. In the first place retrieval medium Ham F10 1X with HEPES was placed, supplemented with SSS and Penicillin / streptomycin was placed in the retrieval reservoir (second reservoir); then semen samples A, B, C and D were placed after having been processed according to WHO2010 Protocol, in the remaining reservoir (first reservoir).

For both examples the procedure to use the device according to the method of the present invention is the following:
Step 1- Medium Ham F10 1X with HEPES (0.6-0.7mililitres) is placed to level the second reservoir. HamF10 1X with HEPES is loaded with a straw-tip Pasteur pipette to enable entering the cuevette; when it is filled membranes are filled with Ham medium which bathes the inner surface and the immediate surface of the semen side.
Step 2 - Immediately afterwards, the semen sample is placed in the first reservoir. The membrane should be completely covered by the sample to be processed; the surface of said membrane must be used to the maximum.
Step 3 - Incubation follows for one hour (it can range from 20 minutes to 60 minutes) in contact with the body, although it can be performed in an oven.
Step 4 - The device is taken out from the incubation state (whether oven or from being in contact with the body); an oven at 36 °C was used for Example 2, and the fist for Example 1. The processed sample is retrieved from the retrieval medium, i.e., from the second reservoir using a syringe and tuberculin needle. This processed sample is ready to be used in artificial insemination.

For semen evaluation before and after retrieval, the WHO 2010 guidelines were observed. To evaluate DNA the (Terminal Transferase dUTP Nick End Labeling) TUNEL Test was applied using a flow cytometer or a fluorescence microscope.

### Results

The results of the instrument show that applying the method of the present invention (MXM) for glass as the material of choice double the percentage of normal shapes (strict morphology) regarding the untreated semen, from 13.3% in the direct method (average of samples D,E,F) of normal shapes to 27.0% (n:3) with the device of the presentdescription. Comparing MXM of the present invention (glass), with swim-up, the present invention achieves an improvement of 50 % (samples D, E, F) in the morphology of the selected spermatozoa. (Table 1)

In the case of the present invention using PET as the chosen material, the percentage of normally shaped spermatozoa tripled compared with the direct method Increasing from 10.3% to 36.0% (for samples A, B, C, D), average values of the results obtained in each test. While in the swim-up case, almost a two-fold value for normal shape spermatozoa is reached, from 11.3% to 18.0% of normal shape applying the method of the present invention. It is worth mentioning that swim-up is the normal basic technique for spermatozoa retrieval in view of high or low complexity artificial insemination, with the disadvantage that the exposure time and manipulation together with centrifugation may lead to DNA alterations.

On the other hand, morphology is evaluated applying Kruger's criteria, WHO. A sample whose Tunnel Test (2) for DNA integrity, yielded a value of 35 % (pathological, since the values of reference must be below 20%) was taken to perform that same study on swim-up and on MXM with PET and glass, from the same sample (sample D). The values obtained, as it is seen in the following table, show a significant improvement when using MXM on PET: the original value decreases in over a third (from 35 to 10), a remarkably significant value. On the other hand, the values of the Tunnel Test for the methods of swim-up as well as MXM on glass of the present invention for sample D, show an increase of said value with a reduction of almost nine times.

Even though it is observed that with the swim-up sample D achieves a decrease in DNA-damaged spermatozoa which is also equal to that found for MXM, the latter method of the present invention yields a sample with spermatozoa of a three-times better morphology for the said sample.

**Table 1: Results of tests in Examples 1 and 2**

| **SAMPLE** | **Present invention in PET** | | **Present invention in GLASS** | | **Swim Up** | | **DIRECT** | |
|---|---|---|---|---|---|---|---|---|
| | MORPHOLOGY | TUNNEL | MORPHOLOGY | TUNNEL | MORPHOLOGY | TUNNEL | MORPHOLOGY | TUNNEL |
| A | 22 | | | | 13 | | 12 | |
| B | 43 | | | | 26 | | 6 | |
| C | 43 | | | | 18 | | 10 | |
| D | 35 | 10 | 24 | 4 | 8 | 3 | 13 | 35 |
| E | | | 31 | | 26 | | 15 | |
| F | | | 26 | | 20 | | 12 | |
| | | | | | | | | |
| | | | | | | | | |
| Increase % of the normal morphology % regarding untreated semen | comparing A,B,C,D it goes from an average 10.3% of normal shape in untreated semen to 35.7%. ***It implies that the* % *of normal shapes are tripled.*** | | comparing D,E,F it goes from 13.3% of average normal shapes in untreated semen to 27.0% normal shapes. ***It implies that normal shapes are doubled.*** | | Comparing A,B,C,D,E,F it goes from 11.3% of average normal shapes of untreated semen to 18.5% It increases less than double of normal shapes. | | 0 | |
| Increase % of the normal morphology % regarding swim-up | comparing A,B,C,D it goes from 16.3% average of normal shapes in swim up to 35.7% with MXM. ***It implies that normal shapes increased 120%.*** | | Comparing D,E,F it goes from 18.0% to *27.0% **this is morphology increase for MXM, of 50% regarding swim up*** | | | | | |
| Percentage of spermatozoa with damaged DNA decreased regarding fresh semen (only Sample D) | | Percentage of damaged spermatozoa decreased to 1/3 regarding sample without treatment | | Percentage of damaged spermatoz oa decreased to 1/10 regarding sample without treatment | | Percentage of damaged spermatozoa decreased to 1/10 regarding sample without treatment | | |

### References:

(1)Aitken RJ, Sawyer D. The human spermatozoon--not waving but drowning. Adv Exp Med Biol 2003;518:85- 98
(2)A Agarwal, SS Allamaneni - Minerva Ginecol, 2004 - clevelandclinic.org
(3)D Mortimer - Human Reproduction, 1991 - ESHRE
(4)K Miki, DE Clapham - Current Biology, 2013 - Elsevier

## Claims

1. A method to separate motile cells from a cell population using a device which comprises a first reservoir and a second reservoir linked by at least a membrane, **characterized in that** said membrane comprises a multiplicity of channels with at least one end that comprises a reduction in diameter towards the interior of said channels, comprising the following steps:
a) placing the culture medium to flood said second reservoir and said channels;
b) contacting the cell population in said first reservoir with a face of said membrane comprising a multiplicity of channels with at least one end having a reduction in their diameter towards the interior of said channels, wherein said first reservoir is filled with a sample of said population of cells to be separated at a level that is lower than the level of said second reservoir, whereby a pressure difference in said reservoirs is generated, so that continuous passing medium to the first reservoir is allowed, and the initial counter current effect is produced, so that said motile cells swim in counter-current for their separation;
c) incubating;
d) retrieving motile cells of a higher percentage of normal morphology from the other side of said membrane.

2. The method to separate motile cells from a cell population of claim 1 wherein said step "c" comprises incubating for a period of between 1 and 90 minutes at 35 - 37ºC.

3. The method according to claim 1, wherein said channels comprise in at least one of their ends a shape selected from the group comprising of conical, paraboloid and hyperboloid shapes.

4. The method according to claim 1, wherein said cells comprises spermatozoa.

5. The method according to claim 1, wherein said channels comprise a first outer diameter of between 50 and 200 µm, and an inner diameter of between 8 and 15 µm.

6. The method according to claim 1, wherein said channels comprises a first outer diameter of between 50 and 200 µm, and a second outer diameter of between 10 and 50 µm and an inner diameter of between 8 and 15 µm.

7. The method according to claim 1, wherein said membrane comprises between 1,000 and 20,000 channels /cm2.

8. The method according to claim 1, wherein the volume of said first reservoir is between 1 and 5ml, and the volume of said second reservoir is between 0.5 and 1.5 ml.

9. The method according to claim 1, wherein said membrane is made of a material selected from the group comprising of glass, PET, metal, ceramics, polymer, carbon fiber and the mixtures or combinations thereof.

10. The method according to claim 4, wherein the spermatozoa with damaged DNA obtained in the second reservoir is decreased, in comparison with the fresh sample, in at least three-fold.

11. The method according to claim 4, **characterized by** increasing the normal morphology of spermatozoa in at least 80% in comparison with the direct method.

## Patentansprüche

1. Ein Verfahren zur Trennung von motilen Zellen aus einer Zellpopulation unter Verwendung eines Vorrichtung, die ein erstes Reservoir und ein zweites Reservoir aufweist, die durch mindestens eine Membran verbunden sind, **gekennzeichnet dadurch, dass** die Membran eine Vielzahl von Kanälen mit mindestens einem Ende umfasst, die eine Verengung des Durchmessers in Richtung des Inneren der Kanäle aufweisen, umfassend die folgenden Schritte:
a) Platzieren des Kulturmediums, so dass dieses in das zweite Reservoir und die Kanäle fließt;
b) Kontaktieren der Zellpopulation im ersten Reservoir mit einer Seite der Membran, die eine Vielzahl von Kanälen aufweist, von denen mindestens ein Ende eine Verengung des Durchmessers in Richtung des Inneren der Kanäle aufweist, wobei das erste Reservoir mit einer Probe der zu trennenden Zellpopulation auf einem Niveau gefüllt ist, das niedriger ist als das Niveau des zweiten Reservoirs, wodurch ein Druckunterschied in den Reservoirs erzeugt wird, sodass ein kontinuierlicher Durchfluss des Mediums in das erste Reservoir erlaubt ist und der anfängliche Gegenstrom-Effekt erzeugt wird, sodass die motilen Zellen im Gegenstrom schwimmen, um getrennt zu werden;
c) Inkubieren;
d) Ernten der motilen Zellen mit einem höheren Prozentsatz normaler Morphologie von der anderen Seite der Membran.

2. Das Verfahren zur Trennung von motilen Zellen aus einer Zellpopulation gemäß Anspruch 1, wobei der Schritt "c" ein Inkubieren über einen Zeitraum von 1 bis 90 Minuten bei 35 °C bis 37 °C umfasst.

3. Das Verfahren gemäß Anspruch 1, wobei die Kanäle an mindestens einem Ende eine Form aufweisen, die aus der Gruppe der konischen, paraboloiden und hyperboloidalen Formen ausgewählt ist.

4. Das Verfahren gemäß Anspruch 1, wobei die Zellen Spermatozoen umfassen.

5. Das Verfahren gemäß Anspruch 1, wobei die Kanäle einen ersten äußeren Durchmesser von 50 bis 200 µm und einen inneren Durchmesser von 8 bis 15 µm aufweisen.

6. Das Verfahren gemäß Anspruch 1, wobei die Kanäle einen ersten äußeren Durchmesser von 50 bis 200 µm, einen zweiten äußeren Durchmesser von 10 bis 50 µm und einen inneren Durchmesser von 8 bis 15 µm aufweisen.

7. Das Verfahren gemäß Anspruch 1, wobei die Membran zwischen 1.000 und 20.000 Kanäle/cm² umfasst.

8. Das Verfahren gemäß Anspruch 1, wobei das Volumen des ersten Reservoirs zwischen 1 und 5 ml und das Volumen des zweiten Reservoirs zwischen 0,5 und 1,5 ml ist.

9. Das Verfahren gemäß Anspruch 1, wobei die Membran aus einem Material hergestellt ist, das aus der Gruppe ausgewählt ist, bestehend aus Glas, PET, Metall, Keramik, Polymer, Kohlenstofffaser und deren Mischungen oder Kombinationen.

10. Das Verfahren gemäß Anspruch 4, wobei die Spermatozoen mit beschädigter DNA, die im zweiten Reservoir erhalten werden, im Vergleich zur frischen Probe um mindestens das Dreifache verringert sind.

11. Das Verfahren gemäß Anspruch 4, **gekennzeichnet durch** eine Erhöhung der normalen Morphologie der Spermatozoen um mindestens 80 % im Vergleich zur direkten Methode.

## Revendications

1. Procédé de séparation de cellules mobiles d'une population cellulaire à l'aide d'un dispositif qui comprend un premier réservoir et un deuxième réservoir reliés par au moins une membrane, **caractérisé en ce que** ladite membrane comprend une multiplicité de canaux dont au moins une extrémité comprend une réduction de diamètre vers l'intérieur desdits canaux, comprenant les étapes suivantes consistant à :
a) placer le milieu de culture pour inonder ledit deuxième réservoir et lesdits canaux ;
b) mettre en contact la population cellulaire dans ledit premier réservoir avec une face de ladite membrane comprenant une multiplicité de canaux dont au moins une extrémité présente une réduction de leur diamètre vers l'intérieur desdits canaux, dans lequel ledit premier réservoir est rempli d'un échantillon de ladite population de cellules à séparer à un niveau qui est inférieur au niveau dudit deuxième réservoir, ce qui génère une différence de pression dans lesdits réservoirs, de manière à permettre un milieu de passage continu vers le premier réservoir, et l'effet initial de contre-courant est produit, de sorte que lesdites cellules mobiles nagent à contre-courant pour leur séparation ;
c) incuber ;
d) récupérer les cellules mobiles d'un pourcentage plus élevé de morphologie normale de l'autre côté de ladite membrane.

2. Procédé de séparation des cellules mobiles d'une population cellulaire selon la revendication 1, dans lequel l'étape « c » comprend l'incubation pendant une période comprise entre 1 et 90 minutes à 35 - 37°C^{.}

3. Procédé selon la revendication 1, dans lequel lesdits canaux comportent à au moins une de leurs extrémités une forme choisie dans le groupe comprenant les formes coniques, paraboloïdes et hyperboloïdes.

4. Procédé selon la revendication 1, dans lequel lesdites cellules comprennent des spermatozoïdes.

5. Procédé selon la revendication 1, dans lequel lesdits canaux comprennent un premier diamètre extérieur compris entre 50 et 200 µm, et un diamètre intérieur compris entre 8 et 15 µm.

6. Procédé selon la revendication 1, dans lequel lesdits canaux comprennent un premier diamètre extérieur compris entre 50 et 200 µm, et un deuxième diamètre extérieur compris entre 10 et 50 µm et un diamètre intérieur compris entre 8 et 15 µm.

7. Procédé selon la revendication 1, dans lequel ladite membrane comprend entre 1 000 et 20 000 canaux /cm².

8. Procédé selon la revendication 1, dans lequel le volume dudit premier réservoir est compris entre 1 et 5 ml, et le volume dudit deuxième réservoir est compris entre 0,5 et 1,5 ml.

9. Procédé selon la revendication 1, dans lequel ladite membrane est constituée d'un matériau choisi dans le groupe comprenant le verre, le PET, le métal, la céramique, le polymère, la fibre de carbone et les mélanges ou combinaisons de ceux-ci.

10. Procédé selon la revendication 4, dans lequel le nombre de spermatozoïdes dont l'ADN est endommagé, obtenus dans le deuxième réservoir, est réduit d'au moins trois fois par rapport à l'échantillon frais.

11. Procédé selon la revendication 4, **caractérisé par** l'augmentation de la morphologie normale des spermatozoïdes d'au moins 80 % par rapport au procédé direct.
